# EUROPEAN PATENT SPECIFICATION

(11) **EP 2 126 136 B1**
(45) Date of publication and mention of the grant of the patent: **02.11.2011**
(21) Application number: 08701643.2
(22) Date of filing: 23.01.2008
(51) Int. Cl.: C12Q 1/68, G01N 33/574, A61P 35/00, C07K 16/18

(54) **DIAGNOSIS OF PROSTATE CANCER**
DIAGNOSE VON PROSTATAKREBS
DIAGNOSTIC DU CANCER DE LA PROSTATE

(30) Priority: 23.01.2007 US 881830 P
(43) Date of publication of application: 02.12.2009
(73) Proprietor: Centre National de la Recherche Scientifique (CNRS), 75016 Paris (FR); Centre Paul Strauss, 67000 Strasbourg (FR); Université Louis Pasteur, U.L.P., 67000 Strasbourg (FR)
(72) Inventor: WASYLYK, Bohdan, F-67400 Illkirch (FR); ZAMBRANO, Alberto, E-28700 Madrid (ES); ABECASSIS, Joseph, 67085 Strasbourg (FR); KLOCKER, Helmut, A-6401 Inzing (AT); SCHALKEN, Jack, A., NL-6524 LH Nijmegen (NL)
(74) Representative: Warcoin, Jacques
(86) International application number: PCT/EP2008/050761
(87) International publication number: WO 2008/090177

(56) References cited:
- JANKE CARSTEN ET AL: "Tubulin polyglutamylase enzymes are members of the TTL domain protein family." SCIENCE (NEW YORK, N.Y.) 17 JUN 2005, vol. 308, no. 5729, 17 June 2005 (2005-06-17), pages 1758-1762, XP002485174 ISSN: 1095-9203 cited in the application -& 17 June 2005 (2005-06-17), XP002485175 Supporting online material Retrieved from the Internet: URL:www.science.org> [retrieved on 2008-06-20]
- DATABASE GENBANK NCBI; Vers. 2 of 12.12.2006 cf. revision history 12 December 2006 (2006-12-12), COLLINS ET AL.: "Tubulin-tyrosine ligase like protein 12" XP002485177 Database accession no. Q14166 -& DATABASE GENBANK NCBI; Revision History for Q14166 XP002485178 Database accession no. Q14166
- SOUCEK KAREL ET AL: "Normal and prostate cancer cells display distinct molecular profiles of alpha-tubulin posttranslational modifications." THE PROSTATE 15 JUN 2006, vol. 66, no. 9, 15 June 2006 (2006-06-15), pages 954-965, XP002485176 ISSN: 0270-4137
- WASYLYK CHRISTINE ET AL: "Tubulin tyrosine ligase like 12 links to prostate cancer through tubulin posttranslational modification and chromosome ploidy.", INTERNATIONAL JOURNAL OF CANCER. JOURNAL INTERNATIONAL DU CANCER 1 DEC 2010 LNKD- PUBMED:20162578, vol. 127, no. 11, 1 December 2010 (2010-12-01), pages 2542-2553, ISSN: 1097-0215
- DATABASE GEO [Online] NCBI 09 March 2006 Retrieved from http:www.ncbi.nlm.nih.gov Database accession no. GDS1746
- DATABASE GEO [Online] NCBI 30 January 2007 Retrieved from http:www.ncbi.nlm.nih.gov Database accession no. GDS2545
- DATABASE GEO [Online] NCBI 17 December 2006 Retrieved from http:www.ncbi.nlm.nih.gov Database accession no. GDS3289

## Description

### TECHNICAL FIELD

The present invention relates to vitro diagnosis methods of prostate cancer from a test biological sample, comprising measuring the expression level of KIAA0153 in said test biological sample, as well as to methods for screening compounds inhibiting KIAA0153 gene expression or biological activity and uses of such KIAA0153 inhibiting compounds.

### BACKGROUND ART

Prostate cancer is a public health problem of major and increasing importance particularly as the European population ages. In the European Union, it accounts for over 35,000 deaths every year, associated with considerable morbidity.

Many patients are either over-treated or under-treated, due to the absence of reliable prognostic markers. A usually used marker for diagnosis is PSA (prostate specific antigen). However, most men with an elevated PSA test turn out not to have cancer; only 25 to 30 percent of men who have a biopsy due to elevated PSA levels actually have prostate cancer. The PSA test is thus not specific enough to screen for prostate cancer and new markers that perform better than PSA thus need to be developed.

In addition, there is currently no satisfying treatment for advanced prostate cancer. Indeed, advanced prostate cancer treatment is usually performed by hormone ablation therapy and/or prostatectomy, which is not satisfactory since advanced prostate cancer invariably progresses to hormone refractrory disease, leading to death in 3-5 years. There is thus also a need for improved prostate cancer treatments.

The tyrosination cycle has an important role in the cell. In particular, tubulin is subjected to a number of post-translational modifications that regulate the function and organization of microtubules (1), which are essential components of the cell cytoskeleton. One of these modifications is the cyclic removal and re-addition of the C-terminal tyrosine of α-tubulin by tubulin carboxypeptidase and tubulin-tyrosine-ligase (TTL), respectively.

Tubulin tyrosination is conserved among eukaryotes (2) and suppression of TTL in mice causes perinatal death (3). Dividing TTL null cells retain a minor pool of tyrosinated tubulin (Tyr-tubulin), whereas postmitotic TTL null neurons lack Tyr-tubulin and display abnormalities related to neurite growth and axonal differentiation. There is evidence that the tyrosination cycle has a role in proliferation and cancer. Inhibition of TTL accelerates cell proliferation (4). TTL is progressively suppressed during tumor progression in animal models, resulting in accumulation of Glu-tubulin (5). Additionally, TTL is frequently lost in human cancers and tubulin detyrosination is associated with tumor aggressiveness and poor prognosis (6-7).

Interestingly, there are about 14 genes coding for proteins containing TTL domains in the human genome (8) suggesting that they have multiple roles, but the physiological function of most of these proteins and the nature of their substrates are still unknown.

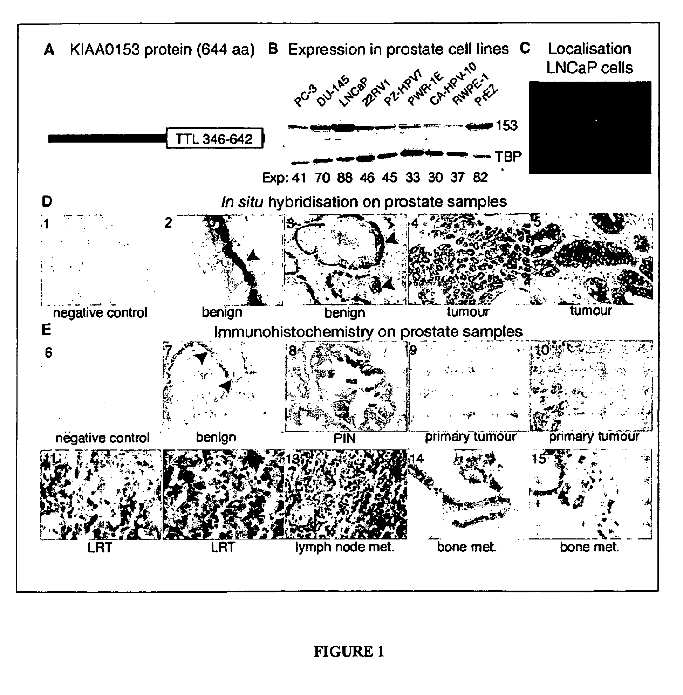

The inventors have performed a phenotypic and functional analysis of one of these novel genes, KIAA0153 (Genbank accession number BC001070). KIAA0153 codes for a 72 KDa protein with a C-terminal TTL domain (Figure 1a, left panel). Tubulin-tyrosine-ligase (TTL), from which the name "TTL domain" originates, is implicated in microtubule functions, such as mitosis and chromosome stability. The presence in KIAA0153 of a TTL-like domain suggests that it may have a similar role in tubulin tyrosination.

Soucek, K. et al., Prostate 66:954-965, 2006 discloses a decreased expression of tubulin tyrosine ligase in prostate cancer cells.

### DESCRIPTION OF THE INVENTION

In contrast, the inventors have surprisingly found that KIAA0153 miss-expression interferes with the normal tubulin tyrosination cycle resulting in changes in the levels of Tyr- and Glu-tubulin. Furthermore it induces mitotic aberrations and chromosome instability. KIAA0153 is expressed in the proliferative cell layer of prostate glands and expression increases with prostate cancer progression. Higher expression in Gleason patterns 4 and 5 suggest that KIAA0153 may be a marker of risk of progression and requirement of more aggressive treatment. According to its expression profile, structure and function, KIAA0153 is thus a new marker of prostate cancer and may represent a novel target for prostate cancer therapy.

The invention thus concerns an in vitro diagnosis method of prostate cancer from a biological tissue sample, comprising measuring the expression level of KIAA0153 in said biological tissue sample.

In one embodiment, said in vitro diagnosis method further comprises measuring the expression level of KIAA0153 in a healthy control biological sample and comparing the expression level of KIAA0153 in both samples. By "healthy sample" is meant a sample from a subject that is not suffering from prostate cancer.

Preferably, prostate cancer is diagnosed when the ratio of the expression level of KIAA0153 in said test biological sample to the expression level of KIAA0153 in said healthy control biological sample is superior to 1.5, advantageously superior to 1.6, 1.7, 1.8, or 1.9.

The results obtained by the inventors show that KIAA0153 is especially highly expressed in metastatic prostate cancer. In an embodiment of an in vitro diagnosis method according to the invention, said method thus further comprises diagnosing metastactic prostate cancer when the ratio of the expression level of KIAA0153 in said test biological sample to the expression level of KIAA0153 in said healthy control biological sample is superior to 2.0.

In a preferred embodiment, said tissue biological sample is a prostate sample. In particular, said prostate sample may be selected from a prostate biopsy, a prostate resection, or a cell line derived from a prostate biopsy or a prostate resection. Preferably, the test sample and the healthy control sample are the same kind of biological tissue sample to allow for a significant comparison. This way, if the test sample is a prostate sample, then the healthy control sample is also preferably a prostate sample.

The measure of KIAA0153 expression level may be performed either at the protein or at the nucleic acid level.

In one embodiment, the expression level of KIAA0153 is determined at the protein level. Various technologies well-known in the art may be used to measure the amount of KIAA0153 protein. Examples of suitable technologies to determine the expression level of KIAA0153 at the protein level include immunohistochemistry, immunoblotting, immunofluorescence, or mass spectrometry, but any person skilled in the art will be able to find other appropriate technologies.

In another embodiment, the expression level of KIAA0153 is determined at the nucleic acid level. In particular, such a measure may be done by measuring the amount of KIAA0153 mRNA or corresponding cDNA. Various technologies well-known in the art may be used to measure the amount of KIAA0153 mRNA or corresponding cDNA. Examples of suitable technologies to determine the expression level of KIAA0153 at the nucleic acid level include in situ hybridization or quantitative PCR, but any person skilled in the art will be able to find other appropriate technologies.

The results obtained by the inventors show that KIAA0153 may be considered as a new therapeutic target for treating prostate cancer. The invention thus also concerns a method for screening compounds for the treatment of prostate cancer by inhibiting KIAA0153 expression, comprising :
a) providing a host cell expressing KIAA0153,
b) providing a test compound,
c) measuring KIAA0153 expression level by said host cell in the presence and in the absence of said test compound, and
d) comparing KIAA0153 expression levels host cells in the presence and in the absence of said test compound.

A host cell expressing KIAA0153 is a cell in which expression of KIAA0153 is detectable. It may be natural or genetically engineered, however, a genetically engineered host cell which has been transfected (transiently or stably) or transformed with an expression vector capable of directing the expression of KIAA0153 is preferred, since it permits to have a controlled expression level of KIAA0153.

As already described for diagnosis methods according to the invention, the expression level of KIAA0153 may be measured either at the protein or nucleic acid level using various technologies.

A test compound will then be considered as inhibiting KIAA0153 expression if KIAA0153 expression is lower in host cells cultured in the presence of said compound than in host cells cultured in the absence of said compound.

The invention also concerns a method for screening compounds for the treatment of prostate cancer by inhibiting KIAA0153 biological activity, comprising:
a) providing a host cell expressing KIAA0153 and tubulin tyrosinase ligase (TTL),
b) providing a test compound,
c) measuring TTL activity in said host cell in the presence and in the absence of said test compound, and
d) comparing TTL activity in host cells in the presence and in the absence of said test compound.

Indeed, KIAA0153 biological activity, which is intended to mean its action in a cell expressing it, may be inhibited either by inhibiting KIAA0153 expression, or by interfering with KIAA0153 action in the cell. The inventors have found that KIAA0153 inhibits TTL tyrosination activity. Thus, a compound inhibiting KIAA0153 biological activity should restore TTL activity.

TTL activity consists in tyrosination of tubulin. Thus, in one embodiment of a method for screening compounds inhibiting KIAA0153 biological activity, TTL activity is measured by measuring incorporation of exogenous tyrosine into tubulin.

This may be done using various technologies easily implemented by a skilled artisan. For instance, the added exogenous tyrosine may be modified to be detectable, in particular by addition of a chemical group that may then be traced, such as a radioactive group, a labelled group (fluorescent, chemiluminescent, etc...). The measure of tyrosine incorporation may also be done by removing tubulin and microtubules and measuring the remaining tyrosine concentration.

Alternatively, inhibition of TTL activity results in an increased concentration in Glu-tubulin. Thus, in another embodiment of a method for screening compounds inhibiting KIAA0153 biological activity, TTL activity is measured by measuring the amount of Glu-tubulin. This measure is easily performed by well-known technologies.

All sorts of compounds may be screened using the methods of screening according to the invention described above. However, some potential inhibitors of KIAA0153 function immediately appear, including antibodies, siRNAs or antisense nucleic acids directed against KIAA0153.

The invention thus further concerns an antibody directed against KIAA0153 protein. Such an antibody may be easily generated using well-know routine technologies for a skilled artisan. The term "antibody" is intended to mean not only an antibody as such but also antibody fragments such as Fab or Fab'2 or ScFv fragments. It may be polyclonal or monoclonal, and may have been further chimerized or humanized using conventional technologies.

The invention also relates to any antibody directed against KIAA0153 protein as described above, as a medicament. Indeed, as a compound able to interfere with KIAA0153 biological activity, such an antibody may be used as a medicament, in particular for treating prostate cancer.

The invention further concerns the use of a KIAA0153 inhibiting compound selected from an antibody, a siRNA or an antisens nucleic acid directed against KIAA0153 for manufacturing a medicament for treating prostate cancer. Any compound able to inhibit KIAA0153 biological activity and/or expression may be used for this purpose.

The disclosure also concerns a method for treating prostate cancer in a patient in need thereof, comprising the administration of a KIAA0153 inhibiting compound.
- The invention also concerns a method for choosing a treatment for a patient suffering from prostate cancer, comprising:measuring the expression level of KIAA0153 in a biological sample of said patient and in a healthy control sample, and
- if KIAA0153 expression level in significantly higher in said patient biological sample than in said healthy control sample, choosing a treatment comprising a KIAA0153 inhibiting compound, or
- else, choosing a treatment comprising another anticancer agent.

Preferably, a treatment comprising a KIAA0153 inhibiting compound is chosen if the ratio of the expression level of KIAA0153 in said test biological sample to the expression level of KIAA0153 in said healthy control biological sample is superior to 1.5, advantageously superior to 1.6, 1.7, 1.8, 1.9 or 2.0.

### DESCRIPTION OF THE FIGURES

**Figure 1****. Expression analysis of KIAA0153. (A)** domain architecture of protein KIAA0153; **(B)** endogenous expression of KIAA0153 in several prostate cell lines. (C) immunocytochemistry staining of endogenous KIAA0153 (LNCaP cells) **Expression of KIAA0153 in prostate specimens. (D)** In situ hybridization on prostate sections; (1) negative control (sense probe); (2-5) antisens KIAA0153-specific probe. The expression of KIAA0153 is localized to the basal layer of prostate glands (photos 2 and 3) and in cancer cells (4 and 5). **(E)** KIAA0153 staining of representative prostate sections: (6) negative control (pre-immune immunoglobulins). The specific antibodies specifically stained the basal layer of normal prostate glands (arrowheads in photo 7). The expression of KIAAO153 is variable and heterogenous throughout the different tumorigenesis stages of prostate cancer: it is present in benign glands (7), in PIN lesions (8), in primary tumors, from moderate to strong staining, photos 9 and 10, respectively, and metastasis. Higher staining scores were found in local recurrencies tumors and metastasis (see Table 1). LRT: local recurrent tumor, hormone insensitive; PrN: prostatic intraepithelial neoplasia. Original magnifications: X400 or X200.

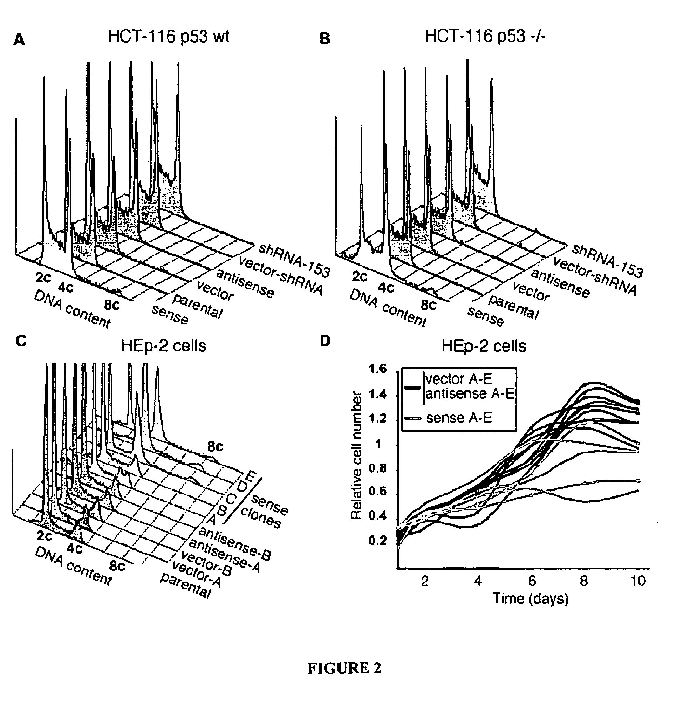

**Figure 2****. Long-term KIAA0153 over-expression induces alterations of the cellular DNA profile**. *FACS profile of propidium-iodide-stained HCT-116 cells;* **(A)** parental HCT-116 p53 wt and stable transfectants cells (see methods); **(B)** set of HCT-116 p53-/- stable transfectants and parental cells. The occurrence of tetraploidy is higher in cells lacking p53 and could only be observed when KIAA0153 was over-expressed (sense cells) *FACS profile and growth curves of the HEp-2 stable clones;* **(C)** representative DNA profile of parental cells, vector, antisense and sense stable clones. In karyotypically unstable HEp-2 cells, the KIAA0153-induced alterations are more dramatic than in HCT-116 cells. It is characterized by elevated G2/M fractions (4c) and the apparition of cells with DNA content 8c. **(D)** growth curves of the total set of stable transfectants (vector, antisense, sense (in lighter grey color), five individual clones of each are represented). We observed that the alterations in the DNA content have dramatic consequences on cell growth. The current chromosome numbers and occurrence of aneuploidy were analyzed by classical karyotyping (see Table 2). The results correlated with DNA profile and suggested a possible role of KIAA0153 in chromosomal instability. We also analyzed clones of unflagged proteins (expressed from pSG5-puro vector; see methods for plasmid info) finding identical results excluding the possibility of artefactual effects of the flag-sequence on the exhibited DNA profile (data not shown). The DNA profile of cells over-expressing other proteins were different to the one induced by KIAA0153 expression.

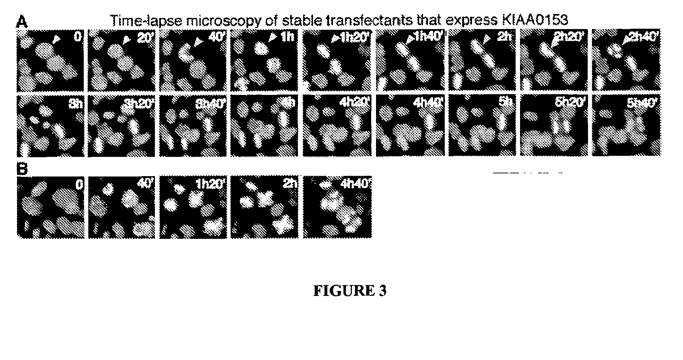

**Figure 3****. Cells over-expressing KIAA0153 exhibit more aberrant multipolar mitoses.** (A) Representative time-lapse course showing three cells undergoing mitoses: two of them executed mitosis correctly (for example see dividing cell near the bottom of the 3-4h time points) but one exhibited an aberrant mitotic spindle and divided into three daughter cells with unequal DNA content. (B) The progression through mitosis of two cells displaying aberrant mitotic spindles are shown; the time for executing mitosis (from first signs of DNA condensation to nuclei separation) is remarkable long (∼ 4 hours) compared to controls (∼ 2h15min-3h). Images were acquired every 20 min during 48 hours (sec Methods). The occurrence of these mitotic aberrations were quantified: 22% of total mitoses were aberrant in cells over-expressing KIAA0153 compared to controls (ranging from 7-9%) (see Table 3).

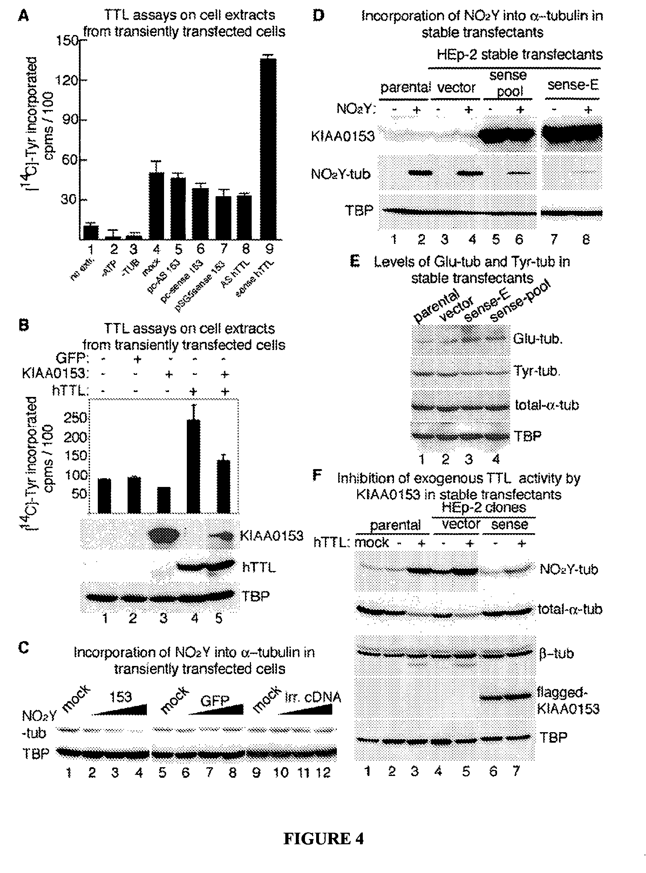

**Figure 4****. KIAA0153 interferes with normal tyrosination of α-tubulin. Classical tubulin** tyrosine ligase (TTL) assays, analysis of α-tubulin pools and incorporation of 3-nitrotyrosine (NO₂Y) onto α-tubulin. (A) TTL assays on cell extracts of transfected HEp-2 cells. 1 to 3 are controls consisting of reaction mixtures without cell extract, ATP or purified tubulin, respectively; the extract of mock-transfected cells (4) revealed the endogenous level of TTL activity; 5: extract of transfected cells with a plasmid harboring antisense KIAA0153 sequence (pc-AS 153); 6 and 7: extracts of cells transfected with pcDNA3-KIAA0153 (pc sense 153) and pSG5-KIAA0153 (pSGspuro-flag-sense 153), respectively. 8 and 9: extracts of cells transfected with antisense version of human TTL (AS hTTL) and sense (sense hTTL), respectively. No TTL activity was observed associated to the expression of KIAA0153 (6 and 7) compared to control (9, human TTL (hTTL)); in fact, the incorporation of tyrosine to tubulin seemed to decrease. **(B)** Inhibition of tubulin tyrosination upon expression of KIAA0153 was confirmed; the expression of KIAA0153 interferes with both endogenous (compare 1 and 3, P=0.015) and exogenous (transfected) human tubulin tyrosine ligase (hTTL) (compare 4 and 5, P=0.0047). **(C)** The incorporation of exogenous NO₂Y is reduced upon transfection of increasing amounts (125, 250 and 500 ng) of KIAA0153-expressing plasmid (left panel: 153) compared to controls (middle and right panels). Irr.cDNA: irrelevant cDNA. **(D)** The levels of nitrotyrosinated-tubulin (NO₂Y-tub are not detectable by this technique as previously reported (lanes -:1, 3, 5 and 7) but the addition of exogenous NO₂Y revealed that nitrotyrosination of α-tubulin is reduced in cells over-expressing KIAA0153 compared to controls (parental cells or stable vector clones; compare lanes 2 and 4 with 6 and 8). KIAA0153 was revealed with polyclonal specific antibody: note moderately low levels of endogenous KIAA0153 of HEp-2 cells. (E) Detyrosinated tubulin (Glu-tubulin) accumulation in cells over-expressing KIAA0153. The levels of Glu-tubulin are higher in stable sense (sense-E or pool of clones, lanes 3 and 4, respectively) compared to controls (lanes 1 and 2); an inverse correlation with respect to tyrosinated-α-tubulin (Tyr-tub) was found. (F) The transfection of hTTL into cells exposed to NO₂Y increased the levels of NO₂Y (lanes +: 3, 5 and 7) but the incorporation of NO₂Y is reduced in cells over-expressing KIAA0153 (compare lane 7 with parental (lane 5) or vector (lane 3)). Interestingly, upon transfection of hTTL, the total levels of c (panel total-α-tub) are higher in the cells over-expressing KIAA0153 (lane 7); this particular effect on α-tubulin was not observed in the absence of exogenous hTTL or controls (lanes -:2, 4, 6, and mock-transfected cells (lane 1)). In the same context, β-tubulin levels remained constant (panel β-tub). We used TBP antibody in all experiments as a protein loading control. Error bars represented standard deviations of triplicate samples of two experiments.

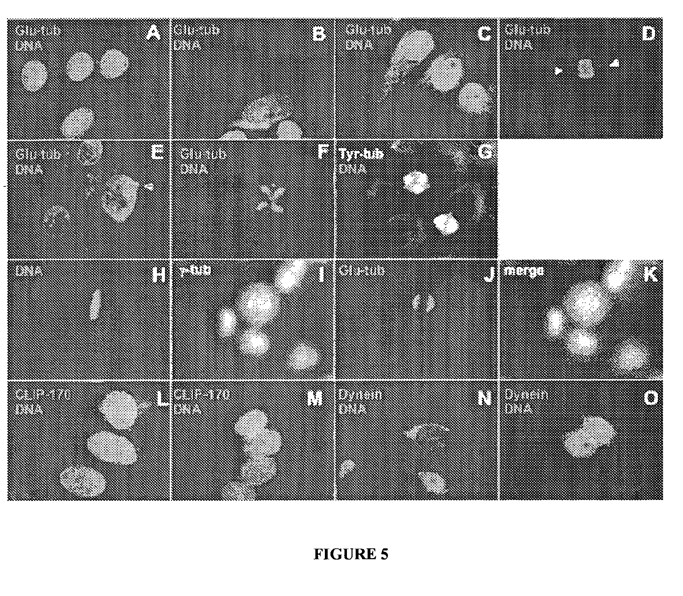

**Figure 5****.** Comparison of the expression of Glu-tubulin, CLIP-170 and dynein between control cells and sense stable transfectants. The staining of Glu-microtubules in parental and control stable HEp-2 cells is less manifest than in cells over-expressing KIAA0153. **(A)** Representative staining of control cells (vector stable transfectant). **(B and C)** Glu-tubulin staining in sense stable cells showing more clear microtubules staining according to their increased expression. **(D)** As previously reported, we found that Glu-tubulin is also localized to the centrioles (arrowheads). **(E)** We observed higher incidence of centrioles accumulation in cells over-expressing KIAA0153 (arrowhead) and many examples of multipolar mitoses **(F). (G)** In the sense transfectants there was an obvious coexistence of mitotic cells exhibiting normal and multipolar mitotic spindles. **(H-K)** Co-localization of γ-tubulin and Glu-tubulin in centrioles. Glu-tubulin antibody also stained the mitotic spindle of sense stable cells. We did not observe significant differences in the distribution of CLIP-170 between control cells of sense stable cells **(L and M, respectively)** or in the distribution of dynein **(N and O)**.

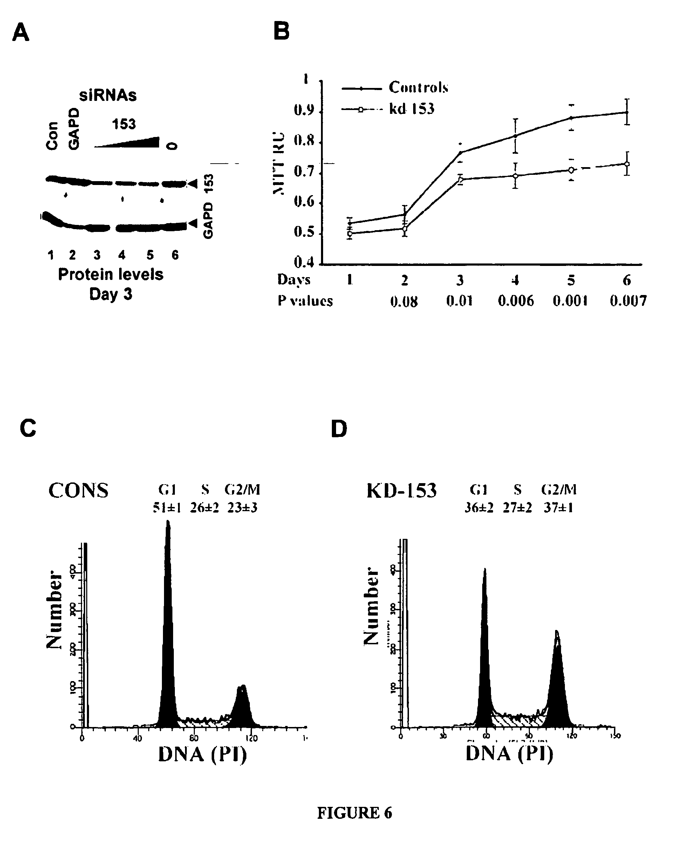

**Figure 6****.** Influence of the knock down of KIAA0153 in the human prostate cancer cell line DU145. **(A)** Analysis of the protein content of DU145 cells (WB) to show the effects of siRNA on KIAA0153 levels. DU145 were split into 6 well plates at 70% confluence, transfected with siRNA and after 48 hours analysed by WB for KIAA0153 and GAPD. Lanes: 1, Control with GFP-siRNA (Qiagen); 2, siRNA GADP (Dharmacon), 3-5, siRNA KIAA153 [25, 50, 100 nM; mixture of #1 (D15633670 Dharmacon, GAGUUCAUCCCCGAGUUUGUU) and #2 (Dharmacon, custom, GGAACGAGCUGUGCUACAAUU)]; 0, control (transfection mix without siRNA). **(B)** Growth curves of DU145 with KIAA0153 kd. Cells at 6K per well (96 well plates, cell number optimised for initial growth) were transfected with Hyperfect and after 1-6 days 5 wells per condition were analysed using the MTT assay (Sigma). The values for the controls (curves 1, 2, 7) and KIAA0153 siRNAs (kd 153, 3-6) were combined to simplify the visualisation of differences. Curves: 1, transfection reagent without siRNA; 2, 50 nM siRNA luc (Dharmacon control), 3, siRNA 153 #1 25 nM; 4, siRNA 153 #1 50 nM; 5, siRNA 153 #2 50 nM; 6, siRNA 153 #1+2 25+25nM; siRNA 153 #1+2 25+25nM; 7, transfection reagent without siRNA. Error bars give the standard deviation of the 5 values. Student test were performed on the values for each day. The differences were "significant" for days 3-6. **(C and D)** Analysis of the DNA content of DU145 cells after KIAA0153 knock down using siRNA. The cells (92K per well, 6 well plates) were transfected with Hyperfect and siRNA Luc (Dharmacon) (CONS) **(C)** or siRNA KIAA0153 **(D)** (#1+2, 25 nM each) and after 48 hours incubation in medium with serum they were incubated for 4 hours in low serum (0.05% serum) and harvested for FACS analysis. The percentages of the cells in the different phases of the cell cycle (G1, S, G2/M) were determined with the CellQuest software. PI = propidium iodide.

### EXAMPLES

### EXAMPLE 1. Phenotypic and functional analysis of KIAA0153

### 1.1 METHODS

### 1.1.1 Cell culture, expression vectors anti transfections.

HEp-2, HCT-116 cells and prostate cell lines were maintained following ATCC (American Type Cell Collection) directions. Transfections were performed by the Calcium-phosphate method. To obtain stable clones, transfected cells were trypsinysed after 48 hours post-transfections (h.p.t.) and splitted into medium containing puromycin (3 mg/ml). The selection medium was replaced every 3 days and clones were isolated after 10-14 days post-transfections (d.p.t.). To obtain double stable transfectants, single stable transfectants (puromycin resistant) were transfected with pBOS H2B-GFP (9) (histone 2B-GFP; BD PharMingen) expression vector and selected with blasticidin (2 mg/ml). Typically, we isolated 5 positive clones by immunofluorescence and western-blotting, per stable transfection. KIAA0153 cDNA clone was obtained from ATCC (MGC-2635 ; IMAGE: 3504490; Genbank number BC001070 ; human placenta). For the expression of flag epitope-tagged proteins, the coding region was PCR-amplified using oligos with XhoI and BclI restrictions sites (CGCCTCGAGATGGAGGCCGAGCGGGGT SEQ ID NO:1, and CGCTGATCACTAGACAAGGCAGGTAACG SEQ ID NO:2) and cloned as a fusion protein into the XhoI/BglII window of pSG5-puro-flag expression vector (10), a modified version of the puromycin-resistant vector pSG5-puro. To obtain the antisense version, we used oligos with XhoI sites. The coding region was also amplified with oligos containing BelI sites and cloned into BglII site of pSG5-puro (without the flag epitope). Alternatively, the coding region amplified with oligos containing EcoRI sites was cloned into pGEM-Teasy (Promega) and pcDNA3.1-(Invitrogen) to obtain antisense and sense versions. The antisense version of KIAA0153 is also referred in the figures as Control-2. Although we did not observe significant differences with respect to vector stable transfectants or parental cells in terms of KIAA0153 expression or functional assays, we maintained it in some experiments to serve as negative control. A clone containing human tubulin tyrosine ligase sequences was purchased to ATCC (MGC-46235; IMAGE:5767758 ; Genbank number BC036819 human brain). The coding region was PCR-amplified with oligos containing the BamHI sites (GATCGGATCCATGTACACCTTCGTGGTACGC SEQ ID NO:3, and CAGGGATCCTCACAGCTTGATGAAGGCA SEQ ID NO:4) and cloned into pSG5-puro-flag expression vector to obtain sense and antisense versions of the gene. Additional vector used were pGFP-Cl (Clontech) for transfection controls, pSuperPuro (Oligoengine) and shRNA153 (shRNA stable down-regulation of KIAA0153 mRNA (mRNA targeted-sequence: GAGUUCAUCCCCGAGUUUG SEQ ID NO:5; oligos were cloned into the pSuperPuro vector). Details on individual plasmids contructs, which were all verified by sequencing, are available upon request. For cell proliferation assay we seeded cells on 96-well plates (3000/well) and use MTT-based colorimetric assay (Chemicon Int.) following manufacturer's directions. When 3-nitrotyrosine (NO₂Y Sigma) was used, it was added to cells 24 hours post-transfection at a final concentration of 400 mM. After 48 hours incubation (72 h.p.t) cells were harvested and processed for immunobloting.

### 1.1.2 Immunoblots.

Cells growing in culture plates were rinsed twice in ice-cold PBS, scraped and centrifuged. The cell pellet was lysed in extraction buffer (50 mM Tris-HCl pH 8, 5 mM EDTA pH 8, 150 mM NaCl, 1% Nonidet-P40, 0,02% sodium azide, 1 mM PMSF, 1X PIC (protease inhibitor cocktail, Amersham). Lysates were sonicated at 0°C for 30 sec and then cleared by centrifugation. Protein concentrations were measured by the Bradford method and 40 mg of total protein were fractionated by 8% SDS-PAGE. Alternatively cell pellets were directly lysated in Laemli buffer, sonicated, boiled and loaded onto the gels. Proteins were transferred to nitrocellulose paper. Western blots were blocked for 1 hour in 5% dried-fat free milk in PBS-Tween 0,05% (TBS-Triton X-100 0.1% for the detection of Glu-tubulin), and then incubated 2 hours with specific antibodies diluted in blocking solution: polyclonal serum against KIAA0153 (1/500), nitrotyrosine (1/1000 ; Upstate Bioteclmology), α-tubulin (1/2000; clone DM1A; Sigma), Tyr-tubulin (1/2000 clone 1A2; Sigma), Glu-tubulin (1/500; Synaptic Systems), β-tubulin (1/2000; Sigma), Tata box protein (TBP) (1/2000 ; IGBMC monoclonal antibodies facility), Flag (1/2000 ; IGBMC monoclonal antibodies facility). Blots were washed and incubated with specific secondary antibodies coupled to HRP (Jackson immunoresearch) and enhance chemiluminiscence reagents (ECL, Pierce) were used for detection.

### 1.1.3 Cell cycle analysis.

Unsynchrosized cells growing exponentially were fixed in 70% ethanol (-20°C), washed and stained with propidium iodide. Detached cell populations were collected and combined with adherent cells for all experiments. FACScalibur cytometer (Becton Dickinson) and CellQuest software were used for acquisition and Modfit software (Verity) for cell cycle analysis.

### 1.1.4 Time-lapse microscopy.

For time-lapse imaging, double stable transfectants (see cell culture and transfections) were analysed on a DMJRE2 inverted microscope (Nikon) with a x40 objective enclosed in a temperature- and CO₂ incubator. Images were acquired with a CCD camera at 20 min intervals and analysed by the Metamorph software.

### 1.1.5 Karyotyping and immunofluorescence.

We performed standard metaphase spreads and Giemsa staining. Typically, 40 metaphase plates of each preparation were analysed. For KIAA0153 immunofluorescence, cells were grown on chambers slides were fixed in Methanol (-20°C) for 5 min and 30 sec in Acetone (-20°C). For the rest of antigens we fixed the cells in Methanol-EGTA 1 mM (-20°C) for 10 min and then 15 min in 4% paraformaldehyde in PBS. Cells were permebealised by a 5 min wash with PBS-0.1% Triton X-100. Cells were blocked with PBS/BSA 1%, and then incubated with specific antibodies diluted in PBS/BSA 1%: polyclonal antibodies raised against KIAA0153 (1/500), Tyr-tubulin (1/500), Glu-tubulin (1/100), polyclonal CLIP-170 (1/250; gift from Jan De Mey (USBS, Strasbourg), dynein (1/200; Sigma), γ-tubulin (from mouse and rabbit origins; Santa Cruz) followed by secondary antibodies coupled to FITC and Cy3 (Jackson Immunoresearch). Slides were mounted with mounting medium containing DAPI (vectashield; Vector). Images were acquired and analysed by the Metamorph software package (Molecular Devices).

### 1.1.6 Tumor specimens, Immunohistochemistry and In situ hybridisation (ISH).

Specimens of local tumor were obtained from untreated patients who underwent radical prostatectomy after tumor diagnosis in a PSA based screening program. In total, 31 benign specimens, 12 PINs, 51 specimens of local tumors, 27 local recurrent tumors (LRT), 8 lymph node and 11 distant metastasis (10 bone, 1 lung) were used. For the analysis of recurrent tumors and metastasis, tissue arrays were employed. A tissue array of locally recurrent tumors was constructed using 2 mm cores from specimens of local resections performed in patients with obstructive symptoms after failure of androgen ablation therapy. These specimens represented "androgen-insensitive" tumors. For the analysis of metastases a lymph node and a distant metastases array was constructed in one paraffin block using three 0.6 mm -cores per case. Immunohistochemistry was performed on 5m-thick formalin-fixed paraffin-embedded tissue sections using polyclonal antibodies raised against KIAA0153 according to a standard Horseradish peroxidase (HRP) immunohistochemistry protocol. After deparaffination in xylenes and rehydration in decreasing ethanol concentrations, tissue sections were heated in citrate buffer pH 6 in a microwave oven for 30 minutes for antigen retrieval. After washing tissue sections were treated for 10 min with 3% H₂O₂ in methanol for inactivation of endogenous peroxidase activity, blocked for 1 hour in Tris-buffered saline solution containing 5 % skim-milk powder and 0.5% Tween20 (TBSTM5) and incubated over night with antibody diluted 1:200 in TBSTM2 at 4°C. For staining, a broad spectrum HRP DAB kit was employed according to the protocol of the manufacturer (Zymed PicTure PLUS Kit, Broad Spectrum, DAB, Zymed). After final washing steps the sections were counterstained with Gui's hematoxylin solution (Sigma) or methyl green (DAKO). Specificity of staining was controlled by including a control antibody (DAKO Cytomation) and stains without first antibody or pre-immune serum. Immunoreactivity was scored according to the Gleason pattern using a 4 point scaling system: 1, no staining, 2, weak staining, 3, intermediate staining, 4, strong staining. For ISH, 5m-thick sections were deparaffinized, rehydrated in decreasing concentrations of ethanol and then air-dried. The slides were then washed in PBS three times for 10 min each. The tissue was permeabilized by proteolytic digestion with proteinase K (6 mg/ml in 50 mM Tris-HCl, pH 7.6) at 50°C for 10 min. The reaction was stopped by rinsing the slides twice in PBS for 5 min each and heating at 92°C for 2 min in a heat block. The slides were placed in PBS for 5 mi HCI 0.2N for 20 mm, PBS for 5 mm, and subsequently dehydrated through washes in graded ethanols, for 5 min each. The tissue was placed in 2XSSC at 70°C for 5 min and then at 92°C for another 5 minutes. Immediately after that, the tissue sections were covered with heat denatured digoxigenin labeled probes diluted 1/50 in hybridisation buffer (50% deionised formamide, 10% dextran sulphate, 1 mg/ml tRNA, 1X Denhardt' s solution, 1X Sait buffer). The siides were incubated at 65°C overnight in a humidity chamber and then, washed four times for 15 min with pre warmed washing solution (1XSSC, 50% formamide, 0.1% tween-20) at 65°C, two times 30 min each with MABT buffer (100 mM maleic acid, 150 mM NaCI, 0.1% Tween-20) at RT and covered with freshly-prepared antibody blocking solution [60% (v/v), goat immunoglobulins 20% (v/v), 2% blocking reagent (Roche)] 1 hour at RT. The sections were covered with goat anti-digoxigenin antibodies coupled to alkaline-phosphatase (Roche) (1/2000 in antibody blocking solution) and incubated 4 hours at 37°C in a humidity chamber. The slides were washed two times 30 min each in MABT buffer, rinsed two times for 10 min at RT with NTMT buffer (100 mM Tris-HCl, ph 9.5, 100 mM NaCl, 50 mM MgCl 0.1% Tween-20). The slides were covered with alkaline phosphatase substrate solution (NBT, BCIP) containing levamisole and incubated for a few hours at RT or overnight at 4°C humidity chamber. Reaction was stopped by washing with PBS/Tween 0.1% and the tissue was counterstained with nuclear fast red (Vector labs) and mounted. Specific sense and antisense probes were synthesized by in vitro transcription ofKIAA0153 expression vector, in the presence of digoxigenin-labelled UTP.

### 1.1.7 Tubulin tyrosine ligase (TTL) assay.

Cells were harvested by a brief trypsin treatment, transferred to tubes containing PBS-10% fetal calf serum and counted, The cells were collected by centrifugation at 950 rpm/5 min/RT. The cells were washed twice with PBS, centrifuged and resuspended in TTL buffer 1X (50 mM Tris-HCl pH 7.4, MgCl 12.5 mM, KCl 100 mM, DTT 1 mM, glycerol 5% Rnase A 50 mg/ml). The cell suspensions were subjected to 3 cycles of freezing-thawing (liquid N₂ - 37°C) and centrifuged at 10000 rpm for 10 min at 4°C. We assayed triplicate samples and, typically, the amount of cell extract corresponding to 3x10 cells. Alternatively, cells were washed, scraped directly in TTL buffer and lysed as indicated above. Identical amount of cell extracts were assayed. The assay mixture was incubated at 37°C for 1 hour and contained the following reagents in addition to the TTL buffer: 4 mM ATP, 200 mg/ml purified rabbit tubulin, 0.5 mCi of L-[U-¹⁴C] Tyrosine (434 mCi/mmol; Amersham). Final reaction volume was 50 ml. The rabbit tubulin was purified by two cycles in an in vitro polymerisation system described by Shelanski et al (11). The reaction was stopped by placing the samples in ice and immediately pipetting 50ml- samples into 1 ml of 10% trichioroacetic acid (TCA) containing 20 mg of unlabelled tyrosine (Sigma). BSA (200 mg) was added as carrier, and the TCA precipitates were kept at 0°C for 30 min. The TCA precipitates were collected on GFC Whatman glass fiber filters. The samples were washed three times with 5 ml of 10% TCA, dried, dissolved in Scillintation liquid cocktail (ReadySafe™ Beckman) and counted in a Beckman scillintation counter.

### 1.1.8 Statistical analyses_{.}

Statistical analyses were performed using Student t-test.

### 1.1.9 KIAA0153 knock down experiments in human prostate cancer cell lines DU145, LnCAP and 22RV1

*Cells were transfected with the Fast Forward Protocol according to the manufacture'rs instructions (Hyperfect, Qiagen).* Western blots: Cells were split into 6 well plates at 70% confluence, and transfected with siRNAs. Control GFP-siRNA (Qiagen); siRNA GADP (Dharmacon); siRNAs KIAA153 [mixture of #1 (D15633670 Dharmacon) and #2 (Dharmacon, custom)]. Cells were transfected with Hyperfect (Qiagen), and incubated for 48 hours in high serum medium (without removing reagent). Cells were scraped into TGKD, proteins were determined by the Pierce (Bradford) method, and 10 µg used for WB with PAb#2089 (KIAA0153) and GAPDH (monoclonal MAb374 Chemicon). Growth curves: DU145 cells at 6K per well (96 well plates, cell number optimised for initial growth) were transfected with Hyperfect and after 1-6 days 5 wells per condition were analysed using the MTT assay (Sigma). *Cell cycle progression: At various time after transfection (48 and 72 hours), the cells were incubated in low serum (4 or 16 hours, respectively), washed twice with PBS, once with PBS + 3 mM EDTA, incubated in 400 µl 0.05% trypsin followed by 2 ml complete medium, centrifuged, washed in PBS, precipitated with EtOH (PBS: EtOH 1: 2 vol: vol), incubated overnight at 4°C, rehydrated in cold PBS, incubated at RT for 2 hours, centrifuged, washed twice with PBS and resuspended in PBS* + *I mg per ml RNAase 1, incubated 2 hours at RT. An equal volume of propidium iodide - TritonX100 was added (0.05 mg*/*ml and 0.1% final concentrations, respectively). The cells were incubated overnight at 4°C in the dark, and analysed by* FACScalibur cytometer (Becton Dickinson). *In general 20K cells were analysed, and cell cycle distribution was estimated with CellQuest software.*

### 1.2 RESULTS

### 1.2.1 Overexpression of KIAA0153 in prostate cancer

KIAA0153 is differentially expressed in head & neck squamous cell carcinoma and prostate cancer (submitted and data not shown) and codes for a 72 KDa protein with a C-terminal TTL domain (Fig. 1A, left panel). KIAA0153 is expressed in prostate cancer cell lines (middle panel), as well as a variety of other cells including mouse embryonic stem cells (data not shown), and is localized in the cytoplasm (right panel). Using in situ hybridization and immunohistochemistry, it was showed that KIAA0153 is expressed in the basal layer of prostate glands (Fig. 1B, photos 1, 3, 6 and 7) and in neoplastic cells (Figure 1B, photos 4, 5, 8-1 5). Expression levels were studied in tumor specimens from 33 patients who underwent radical prostatectomy after tumor diagnosis in a PSA based screening program The obtained results are synthesized in following Table 1:

**Table 1. Scoring of KIAA0153 staining of prostate tissue.**

| Immunoreactivity was scored by a pathologist and stratified according to the Gleason pattern using a 4 point scaling system. 1: no staining, 2: weak staining, 3: intermediate staining, 4: strong staining. Highest scorings were found in local recurrent tumors (LRT) and metastases samples. | | | |
|---|---|---|---|
| **Sample** | **Number** | **Staining Mean Score** | **Remarks** |
| Benign prostate | 31 | 1.0 | |
| PIN | 12 | 2.1 | |
| Primary tumors GP 2-3 | 31 | 1.6 | Predominant |
| | | | GP3 |
| Primary tumors GP 4-5 | 20 | 1.8 | Predominant GP4 |
| Local recurrencies therapy-resistant | 27 | 2.3 | GP 4=1,8 |
| | | | GP 5=2,5 |
| Lymph node metastasis | 8 | 2.6 | |
| Distant metastasis | 11 | 2.3 | 10 bone |
| | | | 1 lung |

KIAA0153 expression in benign epithelial cells was rated 1.0 on average in a 4 point scoring scale. Staining was significantly increased in tumors and there was also a clear association with the Gleason pattern, with 1.6 for Gleason patterns 2 - 3 scored and 1.8 for patterns 4 - 5 scored. Scores also increased with progression, with 2.3 for locally recurrent tumors resected after failure of androgen ablation therapy, 2.6 for lymph node metastasis and 2.3 for distant metastasis. Interestingly, expression in prostate cancer precursor PIN lesions was higher than in the local tumors, almost reaching the levels of recurrent tumors and metastasis (mean score 2.1).

### 1.2.2 Influence of KIAA0153 on microtubule function

The presence of a TTL like domain in KIAA0153 suggests that it may have a role in microtubule functions, such as mitosis and chromosome stability. The karyotypically stable cell line HCT-116, that is widely used to study mitotic events and chromosomal instability and has a normal mitotic checkpoint, was used. The DNA profiles of stable transfectants were analyzed by flow cytometry. Cells over expressing KIAA0153 had a distinct additional peak, corresponding to 8c DNA content (where c is the amount of DNA in a haploid HCT-116 cell) compared to controls (Fig. 2A). Loss of p53 has been reported to synergizes with additional oncogenic events to promote aneuploidy but is not a primary cause of aneuploidy (13). Stable transfectants of HCT116 p53 -/- cells had a higher frequency of 8c cells (compare sense cells in both cell lines).

A synthesis of karyotypic results is presented in following Table 2:

### Table 2. Summary of karyotyping results.

Karyotype analysis was performed on cells derived from single-clones. Chromosome instability was measured as the percentage of cells deviating from modal chromosome number (45 and 74 for HCT- 116 and HEp-2 cells, respectively). Control-2 is a stable transfectant made up by transfecting the antisense version of KIAA0153. Although we did not observe significant differences with respect to vector stable transfectants or parental cells in terms of KIAA0153 expression or in functional assays, we maintained it in this and other experiments to serve as negative control.

| | | parental | vector | control-2 | sense |
|---|---|---|---|---|---|
| HCT-116 p53 wt | modal chrom,# | 45 | 45 | 45 | 45 |
| | % cells 4c or >4c | - | - | - | 10(4c) |
| | % cells deviating from modal chr.# | 25 | 25 | 20 | 35 |
| HCT-116 p53 -/- | modal chrom.# | 45 | 45 | 45 | 45 |
| | % cells 4c or >4c | - | 5(4c) | - | 20(4c) 5(5c) |
| | % cells deviating from modal chr.# | 30 | 35 | 30 | 50 |
| HEp-2 | modal chrom.# | 74 | 74 | 74 | 74 |
| | % Cells 4c or >4c | 5(4c) | - | 5(4c) | 20(4c) |
| | % cells deviating from modal chr.# | 40 | 45 | 40 | 63 |

Analysis of metaphase spreads showed that, compared to controls, clones over-expressing KIAA0153 have higher frequencies of cells with (8c), and the loss of p53 led to an increase in the proportion of cells with 90 chromosomes and the appearance of an addition population with 131 chromosomes (12e). We extended the analysis to karyotypically unstable cells with the purpose of exacerbating this striking phenotype. HEp-2 cells were chosen because they are amenable to stable transfection and synchronisation, and have relatively low levels of endogenous KIAA0153 (see below and data not shown). Individual clones over-expressing KIAA0153 had distinct new peaks with 8c DNA content, and higher G2/M peaks consistent with the presence of overlapping diploid and tetraploid cycles (Fig.2B left panel). Karyotype analysis showed that KIAA0153 overexpression generated a relatively large proportion of cells with 136 chromosome (8c DNA content) (see Table 2). Another consequence of KIAA0153 overexpression is decreased proliferation (Fig.2B, lighter grey curves in the right panel).

Chromosome dynamics in living cells was followed using cells stably expressing histone H2B-GFP [9, see Methods]. Unsynchronised cultures were observed for 48 hours and mitotic events were analyzed. In cells over-expressing KIAA0153 there was a striking high incidence of aberrant mitoses compared to controls. There were metaphase plates with distinct "L" and "X" shapes indicative of aberrations in the mitotic spindle that could yield daughter cells with unequal DNA content (Fig. 3A and following Table 3). The cells with multipolar mitotic spindles took longer to execute anapase (Fig. 3B), which could explain the slower growth rate of these cultures. There was also a higher incidence of apoptosis in cells over-expressing KIAA0153.

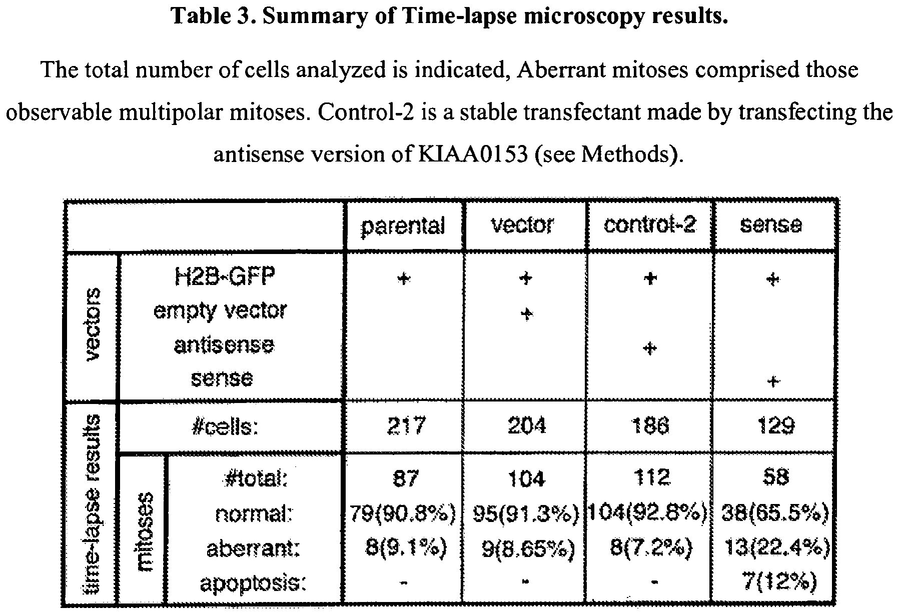

### 1.2.3 Influence of KIAA0153 on TTL activity

Since KIAA0153 has a putative TTL domain, TTL activity in transient and stably transfected cells was studied. Using in vitro assays with cell extracts from transiently transfected cells, it was found that KIAA0153 expression did not increase overall TTL activity, in contrast to expression of human TTL (Fig. 4A), but rather significantly inhibited TTL activity (lanes 6 and 7; Fig. 4B lanes 1 and 3, P=0.015). KIAA0153 also inhibited exogenous co-transfected TTL (Fig. 4B lanes 4 and 5, P=0.047). Tyrosination in vivo was studied by following incorporation of exogenous 3-nitrotyrosine (NO₂Y a natural substrate of TTL. Transient expression of increasing amounts of KIAA0153 plasmid progressively inhibited incorporation (Fig. 4C). KIAA0153 overexpression in stable transfectants also inhibited nitrotyrosinated of α-tubulin (Fig. 4D). In the stable transfectants, we found that there is an increase in endogenous Glu-tubulin in clones expressing KIAA0153, without effects on total α-tubulin levels (Fig. 4E). KIAA0153 expression in a sense clone inhibits the activity of exogenous transfected hTTL and decreased total α-tubulin expression in the absence of changes in β-tubulin or TBP levels (Fig. 4F).

The cellular localisation of Glu-tubulin in the stable clones was also studied. Glu-tubulin is normally preferentially located in a subset of less dynamic microtubules (14-16) as well as in centrioles and mid-bodies, and is absent from astral microtubules (16-17). Tyr-tubulin is found throughout the interphase network and the mitotic spindle. KIAA0153 overexpression did not detectably alter the cellular distribution of the evidently increased levels of Glu-tubulin (Fig 5), and it did not affect microtubule and actin skeleton organization. It also did not affect the cellular distributions of the microtubule tip proteins EB1 (data not shown) and CLIP-170, and the molecular motor dynein (Fig 5).

### 1.2.4 Knock down of KIAA0153 in human prostate cancer cell lines inhibits proliferation

The influence of the knock down of KIAA0153 in human prostate cancer cell lines DU145, LnCAP and 22RV1 was tested using siRNAs directed against KIAA0153.

Results obtained for human prostate cancer line DU145 are presented in Figure 6 and show that the inhibition of KIAA0153 expression results in a decrease of protein levels (Fig. 6A), in an inhibition of cell proliferation (Fig. 6B) and in an accumulation of cells in the G2 phase (Fig. 6C), thus blocking the mitosis process. The results were similar in LnCAP and 22RV1 (data not shown). These results show that KIAA0153 has a role in proliferation and cell cycle progression, and clearly demonstrate the interest of KIAA0153 as a target for prostate cancer therapy.

### 1.3 CONCLUSION

Taken together, our results show that aberrant expression of the novel gene KIAA0153 is associated with prostate cancer progression. It leads to chromosome instability that could result from: inhibition of tubulin tyrosination, increases in the number of centrioles and abnormal multipolar mitoses. Interestingly, Glu-tubulin is normally enriched in centrioles, suggesting that it may have a role in centriole function (18-19) and that KIAA0153 may perturb this function. Furthermore, our preliminary data suggests that KIAA0153 interacts with a centriolar protein (data not shown). KIAA0153 synergises with loss of p53 in the induction of chromosome instability, consistent with it having oncogenic properties (13). KIAA0153 may favour tumorigenesis by increasing chromosome instability, or alternatively by affecting the incorporation of endogenous nitrotyrosine into α-tubulin. Nitrotyrosination of alpha-tubulin in microtubules may act as a "last check point" by triggering apoptosis in aberrant cells (20,21). Expression analysis in benign and malignant prostate tissue revealed a significant increase of expression in tumors and association of expression levels with the progression steps of prostate cancer. Immunoreactivity in tumor specimens increases from untreated primary tumors to local recurrent, androgen-insensitive tumors and metastases. Within the group of primary tumors expression levels were higher in the Gleason pattern 4 and 5 tumor regions as compared to patterns 2 and 3. The presence of the two highest Gleason patterns in a tumor significantly increase the risk of progression suggesting that KIAA0153 levels at the time of initial prostate cancer treatment are related to the risk of progression and requirement of more aggressive treatment. Interestingly, expression in PIN lesion, which are considered prostate cancer precursor lesions, were almost as high as in recurrent disease and metastases, suggesting that upregulation of KIAA0153 expression is an early event of malignant changes occurring in the prostate gland. Highest levels in tumors progressing after escape from androgen ablation therapy and much higher expression in tumors as compared to benign prostate tissue identify the KIAA0153 as a potential target for therapy of prostate cancer after failure of androgen ablation treatment.

Finally, promising results have been obtained by knocking down KIAA0153 expression in human prostate cancer cell lines using siRNA directed against KIAA0153, thus demonstrating the interest of KIAA0153 as a target for prostate cancer therapy.

### BIBLIOGRAPHY

1. Westermann, S. & Weber, K. Post-translational modifications regulate microtubule function. Nat Rev Mol Cell Biol 4,938-47 (2003).
2. Preston, S.F., Deanin, G.G., Hanson, R.K. & Gordon, M.W. The phylogenetic distribution of tubulin:tyrosine ligase. JMolEvol 13, 233-44 (1979).
3. Erck, C. et al. A vital role of tubulin-tyrosine-ligase for neuronal organization. Proc Nati Acad Sci U S A 102, 7853-8 (2005).
4. Mas, C.R., Arregui, C.O., Filiberti, A., Argarana, C.E. & Barra, H.S. Cloning of rat olfactory bulb tubulin tyrosine ligase cDNA: a dominant negative mutant and an antisense
5. Lafanechere, L. et al. Suppression of tubulin tyrosine ligase during tumor growth. J Cell Sci 111 (Pt 2), 171-81 (1998).
6. Mialhe, A. et al. Tubulin detyrosination is a frequent occurrence in breast cancers of poor prognosis. Cancer Res 61, 5024-7 (2001).
7. Kato, C. et al. Low expression of human tubulin tyrosine ligase and suppressed tubulin tyrosination/detyrosination cycle are associated with impaired neuronal differentiation in neuroblastomas with poor prognosis. Int J Cancer 112,365-75 (2004).
   cDNA increase the proliferation rate of cells in culture. Neurochem Res 27, 1453-8 (2002).
8. Janke, C. et al. Tubulin polyglutamylase enzymes are members of the TTL domain protein family. Science 308, 1758-62 (2005).
9. Kanda, T., Sullivan, K.F. & Wahl, G.M. Histone-GFP fusion protein enables sensitive analysis of chromosome dynamics in living mammalian cells. Curr Bio! 8, 377-85 (1998).
10. Nielsen, A.L. et al. Heterochromatin formation in mammalian cells: interaction between histones and HP1 proteins. Mol Cell 7,729-39 (2001).
11. Shelanski, M.L., Gaskin, F. & Cantor, C.R. Microtubule assembly in the absence of added nucleotides. Froc Nati Acad Sci U S A 70, 765-8 (1973).
12. Cavusoglu, N., Brand, M., Tora, L. & Van Dorsselaer, A. Novel subunits of the TATA binding protein free TAFII-containing transcription complex identified by matrix-assisted laser desorption/ionization-time of flight mass spectrometry following one-dimensional gel electrophoresis. Proteomics 3, 217-23 (2003).
13. Duensing, A. & Duensing, S. Guilt by association? p53 and the development of aneuploidy in cancer. Biochem Biophys Res Commun 331, 694-700 (2005).
14. Wehland, J. & Weber, K. Turnover of the carboxy-terminal tyrosine of alpha tubulin and means of reaching elevated levels of detyrosination in living cells. J Ce!! Sci 88 (Pt 2), 185-203 (1987).
15. Webster, D.R., Wehland, J., Weber, K. & Borisy, G.G. Detyrosination of alpha tubulin does not stabilize microtubules in vivo. J CellBiol 111, 113-22 (1990).
16. Kreis, T.E. Microtubules containmg detyrosinated tubulin are less dynamic. Embo J 6, 2597-606 (1987).
17. Gundersen, G.G., Kalnoski, M.H. & Bulinski, J.C. Distinct populations of microtubules: tyrosinated and nontyrosinated alpha tubulin are distributed differently in vivo. Cell 38, 779-89 (1984).
18. Bobinnec, Y. et al. Glutamylation of centriole and cytoplasmic tubulin in proliferating non-neuronal cells. Cell Motu Cytoskeleton 39,223-32 (1998).
19. Bobinnec, Y. et al. Centriole disassembly in vivo and its effect on centrosome structure and function in vertebrate cells. J Cell Biol 143, 1575-89 (1998).
20. Eiserich, J.P. et al. Microtubule dysfunction by posttranslational nitrotyrosination of alpha-tubulin: a nitric oxide-dependent mechanism of cellular injury. Proc NatlAcadSci USA 96, 6365-70 (1999).
21. Idriss, H.T. Three steps to cancer: how phosphorylation of tubulin, tubulin tyrosine ligase and P-glycoprotein may generate and sustain cancer. Cancer Chemother Pharmacol 54, 10 1-4 (2004).

## Claims

1. An in vitro diagnosis method of prostate cancer from a biological tissue sample, comprising measuring the expression level of KIAA0153 in said biological tissue sample.

2. The method of claim 1, further comprising measuring the expression level of KIAA0153 in a healthy control biological sample and comparing the expression level of KIAA0153 in both samples.

3. The method of claim 2, wherein prostate cancer is diagnosed when the ratio of the expression level of KIAA0153 in said test biological sample to the expression level of KIAA0153 in said healthy control biological sample is superior to 1.5.

4. The method of claim 3, further comprising diagnosing metastactic prostate cancer when the ratio of the expression level of KIAA0153 in said test biological sample to the expression level of KIAA0153 in said healthy control biological sample is superior to 2.0.

5. The method of any of claims 1-4, wherein said test biological sample is a prostate sample, preferably a prostate biopsy, a prostate resection, or a cell line derived from a prostate biopsy or a prostate resection.

6. The method of any of claims 1-5 wherein the expression level of KIAA0153 is determined at the protein level, preferably using immunohistochemistry, immunoblotting, immunofluorescence, or mass spectrometry.

7. The method of any of claims 1-5, wherein the expression level of KIAA0153 is determined at the nucleic acid level, preferably using in situ hybridization or quantitative PCR.

8. A method for screening compounds for the treatment of prostate cancer by inhibiting KIAA0153 gene expression, comprising:
a) providing a host cell expressing KIAA0153,
b) providing a test compound,
c) measuring KIAA0153 expression level by said host cell in the presence and in the absence of said test compound, and
d) comparing KIAA0153 expression levels host cells in the presence and in the absence of said test compound.

9. A method for screening compounds for the treatment of prostate cancer by inhibiting KIAA0153 biological activity, comprising:
a) providing a host cell expressing KIAA0153 and tubulin tyrosinase ligase (TTL),
b) providing a test compound,
c) measuring TTL tubulin tyrosination activity in said host cell in the presence and in the absence of said test compound, and
d) comparing TTL tubulin tyrosination activity in host cells in the presence and in the absence of said test compound.

10. The method of claim 9, wherein TTL tubulin tyrosination activity is measured by measuring incorporation of exogenous tyrosine into tubulin or by measuring the amount of Glu-tubulin.

11. An antibody directed against KIAA0153 protein, as a medicament.

12. Use of a KIAA0153 inhibiting compound, selected from an antibody, a siRNA or an antisens nucleic acid directed against KIAA0153, for manufacturing a medicament for treating prostate cancer.

## Patentansprüche

1. In vitro-Verfahren für die Diagnose von Prostatakrebs in einer biologischen Gewebeprobe, umfassend das Messen des Expressionsspiegels von KIAA0153 in der biologischen Gewebeprobe.

2. Verfahren nach Anspruch 1, des Weiteren umfassend das Messen des Expressionsspiegels von KIAA0153 in einer gesunden biologischen Kontrollprobe und das Vergleichen des Expressionsspiegels von KIAA0153 in beiden Proben.

3. Verfahren nach Anspruch 2, wobei Prostatakrebs diagnostiziert wird, wenn das Verhältnis des Expressionsspiegels von KIAA0153 in der biologischen Testprobe zum Expressionsspiegel von KIAA0153 in der gesunden biologischen Kontrollprobe höher als 1,5 ist.

4. Verfahren nach Anspruch 3, des Weiteren umfassend das Diagnostizieren von metastatischem Prostatakrebs, wenn das Verhältnis des Expressionsspiegels von KIAA0153 in der biologischen Testprobe zum Expressionsspiegel von KIAA0153 in der gesunden biologischen Kontrollprobe höher als 2,0 ist.

5. Verfahren nach einem der Ansprüche 1 bis 4, wobei die biologische Testprobe eine Prostataprobe ist, bevorzugt eine Prostatabiopsie-Probe, eine Prostataresektions-Probe oder eine von einer Prostatabiopsie oder Prostataresektion stammende Zelllinie.

6. Verfahren nach einem der Ansprüche 1 bis 5, wobei der Expressionsspiegel von KIAA0153 auf Proteinebene, bevorzugt mittels Immunhistochemie, Immunblotting, Immunfluoreszenz oder Massenspektometrie, bestimmt wird.

7. Verfahren nach einem der Ansprüche 1 bis 5, wobei der Expressionsspiegel von KIAA0153 auf Nucleinsäureebene, bevorzugt mittels in situ-Hybridisierung oder quantitativer PCR, bestimmt wird.

8. Verfahren zum Screenen von Verbindungen für die Behandlung von Prostatakrebs durch Hemmung der Genexpression von KIAA0153, umfassend:
a) Bereitstellen einer Wirtszelle, die KIAA0153 exprimiert,
b) Bereitstellen einer Testverbindung,
c) Messen des Spiegels der KIAA0153-Expression durch die Wirtszelle in Anwesenheit und Abwesenheit der Testverbindung, und
d) Vergleichen der Spiegel der KIAA0153-Expression durch Wirtszellen in Anwesenheit und Abwesenheit der Testverbindung.

9. Verfahren zum Screenen von Verbindungen für die Behandlung von Prostatakrebs durch Hemmung der biologischen Aktivität von KIAA0153, umfassend:
a) Bereitstellen einer Wirtszelle, die KIAA0153 und Tubulintyrosinaseligase (TTL) exprimiert,
b) Bereitstellen einer Testverbindung,
c) Messen der TTL-Tubilintyrosinierungsaktivität in der Wirtszelle in Anwesenheit und Abwesenheit der Testverbindung, und
d) Vergleichen der TTL-Tubilintyrosinierungsaktivität in Wirtszellen in Anwesenheit und Abwesenheit der Testverbindung.

10. Verfahren nach Anspruch 9, wobei die TTL-Tubilintyrosinierungsaktivität durch Messen des Einbaus von exogenem Tyrosin in Tubulin oder durch Messen der Glu-Tubulin-Menge gemessen wird.

11. Gegen KIAA0153-Protein gerichteter Antikörper als Medikament.

12. Verwendung einer KIAA0153-hemmenden Verbindung ausgewählt aus einem Antikörper, einer siRNA oder einer Antisense-Nucleinsäure, die gegen KIAA0153 gerichtet sind, zur Herstellung eines Medikaments für die Behandlung von Prostatakrebs.

## Revendications

1. Procédé de diagnostic *in vitro* du cancer de la prostate à partir d'un échantillon de tissu biologique, comprenant la mesure du taux d'expression de KIAA0153 dans ledit échantillon de tissu biologique.

2. Procédé selon la revendication 1, comprenant en outre la mesure du taux d'expression de KIAA0153 dans un échantillon biologique contrôle sain et la comparaison du taux d'expression de KIAA0153 dans les deux échantillons.

3. Procédé selon la revendication 2, dans lequel le cancer de la prostate est diagnostiqué lorsque le rapport du taux d'expression de KIAA0153 dans ledit échantillon biologique à tester sur le taux d'expression de KIAA0153 dans ledit échantillon biologique contrôle sain est supérieur à 1,5.

4. Procédé selon la revendication 3, comprenant en outre le diagnostic d'un cancer de la prostate métastasé lorsque le rapport du taux d'expression de KIAA0153 dans ledit échantillon biologique à tester sur le taux d'expression de KIAA0153 dans ledit échantillon biologique contrôle sain est supérieur à 2,0.

5. Procédé selon l'une quelconque des revendications 1 à 4, dans lequel ledit échantillon biologique à tester est un échantillon de prostate, de préférence une biopsie de prostate, une résection de prostate ou une lignée cellulaire dérivée d'une biopsie de prostate ou d'une résection de prostate.

6. Procédé selon l'une quelconque des revendications 1 à 5, dans lequel le taux d'expression de KIAA0153 est déterminé au niveau des protéines, de préférence en utilisant l'immunohistochimie, l'immunotransfert, l'immunofluorescence ou la spectrométrie de masse.

7. Procédé selon l'une quelconque des revendications 1 à 5, dans lequel le taux d'expression de KIAA0153 est déterminé au niveau des acides nucléiques, de préférence en utilisant l'hybridation in situ ou une PCR quantitative.

8. Procédé de criblage de composés pour le traitement du cancer de la prostate par inhibition de l'expression du gène KIAA0153, comprenant :
a) la fourniture d'une cellule hôte exprimant KIAA0153,
b) la fourniture d'un composé à tester,
c) la mesure du taux d'expression de KIAA0153 par ladite cellule hôte en présence et en l'absence dudit composé à tester, et
d) la comparaison des taux d'expression de KIAA0153 par les cellules hôtes en présence et en l'absence dudit composé à tester.

9. Procédé de criblage de composés pour le traitement du cancer de la prostate par inhibition de l'activité biologique de KIAA0153, comprenant :
a) la fourniture d'une cellule hôte exprimant KIAA0153 et la tubuline tyrosine ligase (TTL),
b) la fourniture d'un composé à tester,
c) la mesure de l'activité de tyrosination de la tubuline de la TTL dans ladite cellule hôte en présence et en l'absence dudit composé à tester, et
d) la comparaison de l'activité de tyrosination de la tubuline de la TTL dans des cellules hôtes en présence et en l'absence dudit composé à tester.

10. Procédé selon la revendication 9, dans lequel l'activité de tyrosination de la tubuline de la TTL est mesurée en mesurant l'incorporation de tyrosine exogène dans la tubuline ou en mesurant la quantité de Glu-tubuline.

11. Anticorps dirigé contre la protéine KIAA0153, en tant que médicament.

12. Utilisation d'un composé inhibant KIAA0153, choisi parmi un anticorps, un siRNA ou un acide nucléique antisens dirigé contre KIAA0153, pour la fabrication d'un médicament destiné au traitement du cancer de la prostate.


### SEQUENCE LISTING

<110> Centre National de la Recherche Scientifique (CNRS)
   Centre Paul STRASS
   Université Louis Pasteur
   WASYLYK, Bohdan
   ZAMBRANO, Alberto
   ABECASSIS, Joseph
   KLOCKER, Helmut
   SCHALKEN, Jack A.
<120> Diagnosis of prostate cancer
<130> 352443 / D25152
<150> US 60/881,830 <151> 2007-01-23
<160> 5
<170> PatentIn version 3.3
<210> 1
   <211> 27
   <212> DNA
   <213> artificial sequence
<220>
   <223> PCR primer
<400> 1
   cgcctcgaga tggaggccga gcggggt 27
<210> 2
   <211> 28
   <212> DNA
   <213> artificial sequence
<220>
   <223> PCR primer
<400> 2
   cgctgatcac tagacaaggc aggtaacg 28
<210> 3
   <211> 31
   <212> DNA
   <213> artificial sequence
<220>
   <223> PCR primer
<400> 3
   gatcggatcc atgtacacct tcgtggtacg c 31
<210> 4
   <211> 28
   <212> DNA
   <213> artificial sequence
<220>
   <223> PCR primer
<400> 4
   cagggatcct cacagcttga tgaaggca 28
<210> 5
   <211> 19
   <212> RNA
   <213> Homo sapiens
<220>
   <223> KIAA0153 mRNA targeted-sequence
<400> 5
   gaguucaucc ccgaguuug 19